# DEMANDE DE BREVET EUROPEEN

(11) **EP 1 941 847 A1**
(43) Date de publication de la demande: **09.07.2008**
(21) Numéro de dépôt: 07100158.0
(22) Date de dépôt: 05.01.2007
(51) Int. Cl.: A61F 2/44

(54) **Prothèse expansible/ désexpansible d'ecartement de corps vertébraux**

(71) Demandeur: ORTHOPAEDIC & SPINE DEVELOPMENT, 84911 Avignon Cédex 9 (FR)
(72) Inventeur: Boulot, Jacques, 31400, Toulouse (FR)
(74) Mandataire: Schmitz, Jean-Marie

(57) **Abrégé**

La présente invention concerne une prothèse expansible/désexpansible d'écartement de corps vertébraux comprenant trois parties, la première partie étant constituée d'un **corps de prothèse** (1), la seconde partie étant constituée par un **corps de translation** (2) complémentaire du corps de prothèse et la troisième partie étant constituée d'un **poussoir de réglage** (3), ledit poussoir de réglage pouvant être engagé dans le corps de prothèse et dans le corps de translation.

## Description

La présente invention concerne une prothèse déformable expansible/ désexpansible in situ d'écartement de corps vertébraux décrite selon la revendication 1.

Le domaine technique auquel se rapporte cette invention concerne le domaine chirurgical, notamment le traitement d'individus souffrant d'une dégénérescence discale au niveau des vertèbres cervicales ou lombaires, ou thoraciques.

L'Etat de la technique est constitué par la demande de brevet européen EP 05108139 décrivant une prothèse déformable d'écartement de corps vertébraux, constituée d'un corps de prothèse et d'un poussoir de réglage engagé dans ledit corps de prothèse, ce corps de prothèse comportant une partie supérieure et une partie inférieure, ces deux parties étant solidaires l'une de l'autre sur une partie de la longueur du corps de prothèse et étant espacées l'une de l'autre par une fente s'étendant de la partie solidarisée et débouchant à l'une des extrémités du corps et traversant le corps de prothèse sur toute sa largeur, le poussoir de réglage étant engagé dans une ouverture pratiquée dans ladite partie solidarisée et s'étendant dans ladite fente, la position axiale du poussoir de réglage par rapport au corps de prothèse étant réglable pour régler l'écartement entre les parties non solidarisées du corps de prothèse, et l'axe longitudinal de ladite ouverture et du poussoir de réglage étant incliné par rapport à l'axe longitudinal central du corps de prothèse, les deux axes restant généralement dans un plan sagittal du corps de prothèse (1).
Le brevet DE 4416605 divulgue une prothèse intervertébrale déformable constituée d'un corps de prothèse et d'un poussoir engagé dans le corps de prothèse.
Le brevet US 6454807 divulgue un corps de prothèse constitué par deux parties distinctes encastrables et d'un poussoir engagé dans le corps de prothèse
Le brevet EP 1138285 divulgue un corps de prothèse formé en une seule partie.

L'**Etat de la Technique le plus proche** est le document EP 05108139 car il divulgue une prothèse intervertébrale déformable constituée d'un corps de prothèse et d'un poussoir de réglage engagé dans le corps de prothèse.
La **différence** entre la présente invention et cet Etat de la technique le plus proche est la coopération du corps de prothèse avec un corps de translation, ledit corps de translation pouvant être déplacé par translation horizontale dans l'axe longitudinal central du corps de prothèse sous l'action du poussoir de réglage. La prothèse expansible/désexpansible in situ de la présente invention est à géométrie longitudinale variable.
L'**effet technique** de cette différence est que l'agrandissement de la surface de la prothèse permet de doubler la superficie de support de l'implant sur les corps vertébraux, ceci permettant de corriger avec précision l'angle de lordose et/ou de cyphose entre deux vertèbres et d'offrir une excellente surface d'appui au corps de prothèse venant en contact avec la vertèbre située directement sous la prothèse. L'agrandissement de la surface de la prothèse permet aussi une augmentation (un doublement au moins) du volume de greffon contenu dans l'implant.
Le **problème technique** objectif à résoudre par la présente invention consiste en la fourniture d'un dispositif alternatif pour améliorer le traitement chirurgical d'un patient soufrant d'une lordose et/ou d'une cyphose.
La **solution** de ce problème est de faire coopérer un corps de prothèse avec un corps de translation, ledit corps de translation pouvant être déplacé par translation horizontale dans l'axe longitudinal central du corps de prothèse sous l'action du poussoir de réglage. La solution résout ce problème.

L'homme du métier n'aurait pas reconnu ce problème et ne l'aurait pas résolu de la manière indiquée sur base de la totalité de l'art antérieur sans faire preuve d'activité inventive car l'Etat de la technique décrit uniquement des prothèses à géométrie longitudinale invariable, càd qu'un poussoir est introduit dans le corps de prothèse provoquant un effet d'inclinaison de la partie supérieure et/ou de la partie inférieure du corps de prothèse de D1, alors que dans la présente invention l'introduction du poussoir aura une répercussion principalement sur l'agrandissement de la superficie de support de la prothèse par translation/ coulissement du corps de translation.

L'agrandissement de la superficie de support de la prothèse par translation/ coulissement du corps de translation de la présente invention engendre ainsi un effet inattendu et surprenant de stabilité de la prothèse et de correction d'une lordose et/ou d'une cyphose.

Ainsi, l'homme du métier n'aurait pas utilisé l'enseignement de l'Etat de la technique le plus proche en combinaison avec les autres documents de l'Etat de la technique car il n'aurait trouvé aucune indication lui suggérant de déplacer un corps de translation par translation horizontale dans l'axe longitudinal central du corps de prothèse sous l'action du poussoir de réglage.

La présente invention a pour **but** de proposer une prothèse qui soit de conception simple de sorte à être mise facilement en oeuvre et qui épouse le plus parfaitement possible la forme des corps vertébraux inférieurs et supérieurs de sorte à rectifier de manière optimale une lordose et/ou une cyphose et ainsi d'améliorer le traitement chirurgical. L'expansion de la prothèse se fait in situ entre les disques vertébraux. L'expansion de la prothèse assure une large surface de contact entre le greffon et l'os une fois la prothèse en position ouverte.

L'invention est décrite ci-après à l'aide de références aux dessins ci-joints dans lesquels :

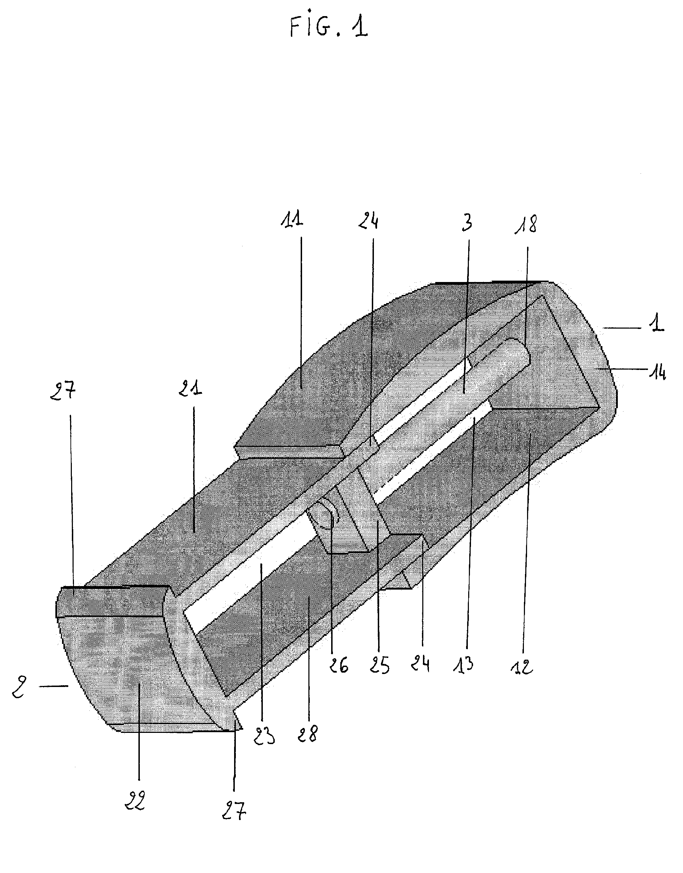

La figure 1 représente une vue en perspective de la prothèse d'écartement de corps vertébraux en position ouverte.

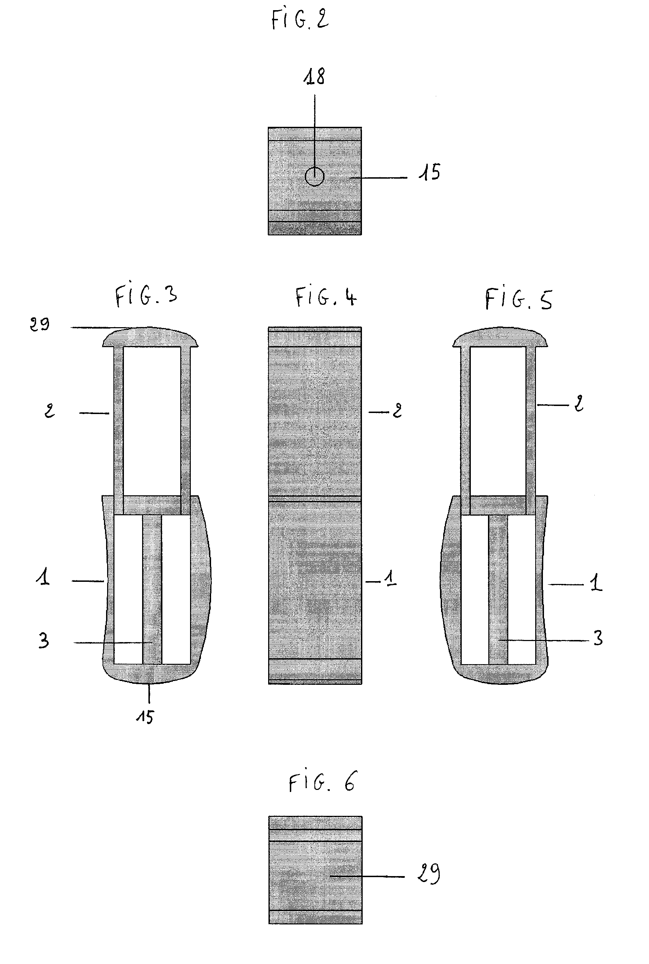

La figure 2 représente une vue de face de la partie postérieure du corps de prothèse (1) en position ouverte.
La figure 3 représente une vue de coté d'une prothèse d'écartement de corps vertébraux en position ouverte.
La figure 4 représente une vue de dessus de la prothèse d'écartement de corps vertébraux en position ouverte.
La figure 5 représente une vue de coté d'une prothèse d'écartement de corps vertébraux en position ouverte.
La figure 6 représente une vue de face de la partie postérieure du corps de translation (2) en position ouverte.

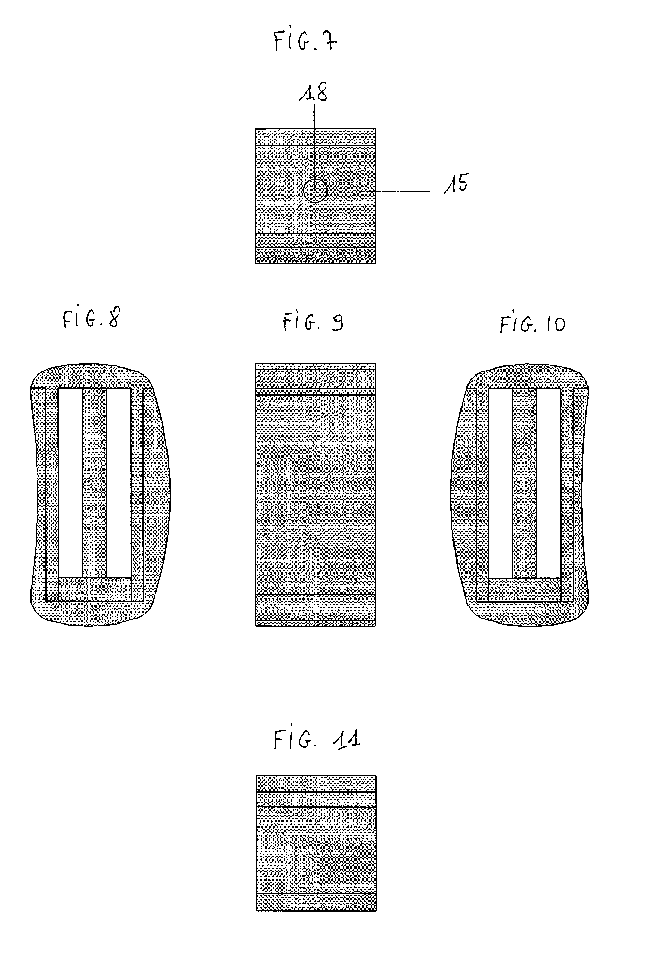

La figure 7 représente une vue de face de la partie postérieure du corps de prothèse (1) en position fermée.
La figure 8 représente une vue de coté d'une prothèse d'écartement de corps vertébraux en position fermée.
La figure 9 représente une vue de dessus de la prothèse d'écartement de corps vertébraux en position fermée.
La figure 10 représente une vue de coté d'une prothèse d'écartement de corps vertébraux en position fermée.
La figure 11 représente une vue de face de la partie postérieure du corps de translation (2) en position fermée.

Conformément à l'invention le but est atteint grâce à une prothèse expansible/ désexpansible in situ d'écartement de corps vertébraux comprenant trois parties, la première partie étant constituée d'un **corps de prothèse** (1), la seconde partie étant constituée par un **corps de translation** (2) complémentaire du corps de prothèse (1) et la troisième partie étant constituée d'un **poussoir de réglage** (3). Le poussoir de réglage peut être engagé dans le corps de prothèse (1) et dans le corps de translation (2), ledit corps de prothèse (1) a une forme en U, comprend un premier bras (11), un second bras (12) et une partie solidarisée (14), ces deux bras (11 et 12) étant reliés par la partie solidarisée (14), ledit corps de translation (2) a une forme en U et est constitué par deux éléments horizontaux parallèles (21 et 28) et une partie solidarisée (22), lesdits éléments (21 et 28) étant reliés par la partie solidarisée (22), ces éléments (21 et 28) étant espacés l'un de l'autre par une fente (23) s'étendant de la partie solidarisée (22) et débouchant à un élément de maintien (25) du corps de translation (2), ladite fente (23) traversant le corps de translation (2) sur toute sa largeur et sa longueur, l'extrémité libre (24) des éléments (21 et 28) étant reliée de manière solidaire par cet élément de maintien (25) qui est lui-même muni d'une ouverture (26), ledit poussoir de réglage (3) étant engagé dans une ouverture (18) pratiquée dans ladite partie solidarisée (14) du corps de prothèse (1) et s'étendant dans une fente (13) pour s'engager dans l'ouverture (26) de l'élément de maintien (25) du corps de translation (2), permettant le déplacement par translation horizontale du corps de translation (2) dans l'axe longitudinal central du corps de prothèse (1) sous l'action du poussoir de réglage (3).

La partie solidarisée (22) du corps de translation (2) fait verticalement saillie (27) par rapport aux éléments horizontaux parallèles (21). Le corps de prothèse (1) comprend une **partie postérieure** (15) convexe. Le corps de translation (2) comprend une **partie postérieure** (29) convexe. Les fentes (13 et 23) sont de forme rectangulaire. Les bras (11 et 12) du corps de prothèse (1) peuvent soit posséder des lumières alignées longitudinalement et des crans transversaux en forme de dents, soit ils peuvent être pleins (càd sans orifice, sans lumière) et lisses. Le premier bras (11) du corps de prothèse (1) est convexe. Le second bras (12) du corps de prothèse (1) est concave.

Le poussoir de réglage (2) est de forme allongée cylindrique et comprend une première partie servant de butée et une deuxième partie constituée par un pas de vis. Le poussoir de réglage (2) peut soit être muni de lumières, soit être plein (càd sans orifice, sans lumière).

La prothèse est réalisée en matière bio implantable (polymère, titane, alliages, acier inox, mixte métal-polymère), en matière minérale ou synthétique par exemple en PEEK (poly ester ethyl carbon), ou en tout autre matière acceptant les contraintes. La prothèse s'adapte à la morphologie des plateaux vertébraux par déformation tridimensionnelle (flexion et torsion) sous l'action simultanée d'un poussoir de réglage, du corps de prothèse, du corps de translation et des corps vertébraux. La déformation principale permet d'obtenir un fort effet lordosant. La déformation de la prothèse permet de supprimer l'impaction de la prothèse dans les corps vertébraux. La prothèse peut être désexpandue, remis en position initiale, refermée in situ avant remplissage par le substitut osseux. La prothèse est implantable par voie gauche ou droite, antérieure ou postérieure ou voie indifférente. La prothèse fonctionne dans les deux sens : ouverture et fermeture grâce au poussoir de réglage muni d'un système de vis.

La mise en mouvement du poussoir de réglage se fait au moyen d'un ancillaire (dispositif de mise en place d'un écarteur de corps vertébraux) approprié. La prothèse est implantable par voie endoscopique ou par voie à ciel ouvert (open surgery).

## Revendications

1. Prothèse d'écartement de corps vertébraux comprenant trois parties, la première partie étant constituée d'un **corps de prothèse** (1), la seconde partie étant constituée par un **corps de translation** (2) complémentaire du corps de prothèse (1) et la troisième partie étant constituée d'un **poussoir de réglage** (3), ledit poussoir de réglage pouvant être engagé dans le corps de prothèse (1) et dans le corps de translation (2),
ledit corps de prothèse (1) ayant une forme en U, comprenant un premier bras (11), un second bras (12) et une partie solidarisée (14), ces deux bras (11 et 12) étant reliés par la partie solidarisée (14),
ledit corps de translation (2) ayant une forme en U, étant constitué par deux éléments horizontaux parallèles (21 et 28) et une partie solidarisée (22), lesdits éléments (21 et 28) étant reliés par la partie solidarisée (22), ces éléments (21 et 28) étant espacés l'un de l'autre par une fente (23) s'étendant de la partie solidarisée (22) et débouchant à un élément de maintien (25) du corps de translation (2), ladite fente (23) traversant le corps de translation (2) sur toute sa largeur et sa longueur, l'extrémité libre (24) des éléments (21 et 28) étant reliée de manière solidaire par cet élément de maintien (25) qui est lui-même muni d'une ouverture (26),
ledit poussoir de réglage (3) étant engagé dans une ouverture (18) pratiquée dans ladite partie solidarisée (14) du corps de prothèse (1) et s'étendant dans une fente (13) pour s'engager dans l'ouverture (26) de l'élément de maintien (25) du corps de translation (2), permettant le déplacement par translation horizontale du corps de translation (2) dans l'axe longitudinal central du corps de prothèse (1) sous l'action du poussoir de réglage (3).

2. Prothèse selon la revendication 1, dans laquelle la partie solidarisée (22) du corps de translation (2) fait verticalement saillie (27) par rapport aux éléments horizontaux parallèles (21)

3. Prothèse selon la revendication 1, dans laquelle le corps de prothèse (1) comprend une partie postérieure (15) convexe.

4. Prothèse selon la revendication 1, dans laquelle le corps de translation (2) comprend une partie postérieure (29) convexe.

5. Prothèse selon la revendication 1, dans laquelle les fentes (13 et 23) sont de forme rectangulaire.

6. Prothèse selon la revendication 1, dans laquelle les bras (11 et 12) du corps de prothèse (1) possèdent des lumières alignées longitudinalement et des crans transversaux en forme de dents.

7. Prothèse selon la revendication 1, dans laquelle les bras (11 et 12) du corps de prothèse (1) sont pleins et lisses.

8. Prothèse selon la revendication 1, dans laquelle le premier bras (11) du corps de prothèse (1) est convexe.

9. Prothèse selon la revendication 1, dans laquelle le second bras (12) du corps de prothèse (1) est concave.

10. Prothèse selon la revendication 1, dans laquelle ledit poussoir de réglage (2) est de forme allongée cylindrique et comprend une première partie servant de butée et une deuxième partie constituée par un pas de vis.

11. Prothèse selon la revendication 10, dans laquelle ledit poussoir de réglage (2) est muni de lumières.

12. Prothèse selon la revendication 10, dans laquelle ledit poussoir de réglage (2) est plein.
